# EUROPEAN PATENT SPECIFICATION

(11) **EP 3 612 140 B1**
(45) Date of publication and mention of the grant of the patent: **01.05.2024**
(21) Application number: 18787294.0
(22) Date of filing: 18.04.2018
(51) Int. Cl.: A61F 2/89, A61F 2/844, A61F 2/962, A61L 31/10, A61F 2/915, A61F 2/966

(54) **DEPLOYABLE STENTS AND RELATED DEVICES, AND SYSTEMS**
ENTFALTBARE STENTS UND ZUGEHÖRIGE GERÄTE UND SYSTEME
STENTS DÉPLOYABLES ET DISPOSITIFS ET SYSTÈMES ASSOCIÉS

(30) Priority: 21.04.2017 US 201762488405 P
(43) Date of publication of application: 26.02.2020
(60) Divisional application: 24166815.1
(73) Proprietor: Merit Medical Systems, Inc., South Jordan, UT 84095 (US)
(72) Inventor: ELLER, Zeke, Plano Texas 75025 (US); ROBINSON, Thomas Patrick, Addison Texas 75001 (US); ELWOOD, Bryan K., Arlington Texas 76014 (US)
(74) Representative: CSY London
(86) International application number: PCT/US2018/028107
(87) International publication number: WO 2018/195160

(56) References cited:
- US-A- 6 015 429
- US-A1- 2001 004 696
- US-A1- 2005 027 345
- US-A1- 2006 030 923
- US-A1- 2008 288 043
- US-A1- 2010 222 864
- US-A1- 2011 004 290
- US-A1- 2012 271 408
- US-A1- 2012 310 363
- US-A1- 2013 116 771
- US-A1- 2014 018 905
- US-A1- 2015 342 764
- US-A1- 2017 079 820
- US-B1- 6 270 524
- US-B1- 6 613 079
- US-B2- 7 335 224

## Description

### RELATED APPLICATIONS

This application claims priority to United States Provisional Application No. 62/488,405, filed on April 21, 2017 and titled "DEPLOYABLE STENTS AND RELATED DEVICES, SYSTEMS, AND METHODS,".

### TECHNICAL FIELD

The present disclosure relates generally to the field of medical devices. More particularly, some embodiments relate to stents that can be deployed within a passageway of a patient. Related devices, and systems are also disclosed.

### BRIEF DESCRIPTION OF THE PRIO ART

US2010/222864 discloses a stent designed for catheter delivery to a target neurovascular site via a tortuous path, in a contracted state, and deployment at the target site, in an expanded state.

US2015/342764 mentions tubular casting processes, such as dip-coating, to form substrates from polymeric solutions which may be used to fabricate implantable devices such as stents.

US2014/018905 is related to a stent to be implanted in a living body is formed substantially as a tube having a form in which plural wavy annular members are arranged adjacent to each other in the axial direction of the stent, with the axially adjacent wavy annular members connected to each other.

US2012/310363 teaches a stent comprised of a scaffolding structure having components configured to allow at least a portion of the stent to decrease in diameter in response to an axial force applied to the stent.

US7335224 discloses a retention system for retaining a stent graft onto a deployment device. The retention system includes a release wire to engage a portion of the exposed stent and to retain the exposed stent in a capsule. The release wire prevents removal of the capsule from the exposed stent through a retention loop.

### STATEMENT OF INVENTION

The invention is according to the appended claims.

### BRIEF DESCRIPTION OF THE DRAWINGS

The written disclosure herein describes illustrative embodiments that are nonlimiting and non-exhaustive. Reference is made to certain of such illustrative embodiments that are depicted in the figures, in which:
FIG. 1 is a perspective view of stent in a compressed state.
FIG. 2 is an alternative perspective view of the stent of FIG. 1 in a compressed state.
FIG. 3 is a perspective view of the stent of FIG. 1 in an expanded state.
FIG. 4 is a perspective view of a stent according to another embodiment.
FIG. 5 is a perspective view of a stent according to another embodiment.
FIG. 6 is a side view of a stent according to another embodiment.
FIG. 7 is a schematic that depicts a tracheobronchial tree of a patient.
FIG. 8 is a perspective view of a stent delivery device.
FIG. 9 is an exploded perspective view of the stent delivery device of FIG. 8.
FIG. 10 is a cross-sectional side view of the stent delivery device of FIG. 8.
FIG. 11 is a cross-sectional perspective view of the stent delivery device of FIG. 8.
FIG. 12 is a cross-sectional view of the stent delivery device through plane 12-12 in FIG. 8.
FIG. 13 is a perspective view of the stent delivery device of FIG. 8 with the outer sheath in a retracted state.
FIG. 14 is a side view of the stent delivery device of FIG. 8 with the outer sheath in a retracted state (and the stent removed to expose certain components).
FIG. 15 is a cross-sectional side view of a portion of the stent delivery device of FIG. 8 in a first configuration.
FIG. 16 is a cross-sectional side view of a portion of the stent delivery device of FIG. 8 in a second configuration.
FIG. 17 is a cross-sectional side view of a portion of the stent delivery device of FIG. 8 in a third configuration.

### DETAILED DESCRIPTION

In some instances, cavities or passageways within a patient may be partially or fully blocked or obstructed, thereby disrupting the flow of fluid (e.g., air or liquid) within the cavity or passageway.

For example, in some embodiments, a portion of an airway may collapse, be damaged, and/or become partially blocked, thereby disrupting, decreasing, or threatening to decrease the flow of air through the passageway. Stents may be used to open, unblock, and/or otherwise support a portion of an airway. Such support may increase the flow of air through an airway.

In some instances, a portion of a lung may collapse or become partially blocked. For example, stricture(s) may be created by malignant neoplasms within the lung of a patient. In such circumstances, it may be desirable to implant a stent into the blocked or collapsed passageway, thereby allowing for (or increasing) the flow of air through the stricture. The stent may be disposed at any suitable position within the respiratory tract. Some embodiments disclosed herein enable the placement of a stent within a portion of a collapsed, damaged, or blocked airway that is distal of either the right main bronchus or the left main bronchus of a patient. For instance, in some circumstances, it may be advantageous to place a stent within a left upper lobe of the lung. In some embodiments, the stent may be disposed at other locations within the respiratory tract of the patient. For example, in some embodiments, the stent is disposed within the right main bronchus or the left main bronchus.

Thus, some embodiments disclosed herein may be used to open, support, and/or unblock a portion of the respiratory tract, such as a portion of the respiratory tract within the bronchi or the lungs. Devices, and systems, for implanting a stent are also disclosed. Stated differently, some embodiments may facilitate or enable the placement of a stent within a portion of a passageway or cavity of a patient. While specific reference is made to the placement of a stent within the respiratory tract of a patient, in some embodiments, the stent may be placed in other locations, such as within the vasculature or biliary tract of the patient.

The components of the embodiments as generally described and illustrated in the figures herein can be arranged and designed in a wide variety of different configurations. Thus, the following more detailed description of various embodiments, as represented in the figures, is not intended to limit the scope of the present disclosure, but is merely representative of various embodiments. While various aspects of the embodiments are presented in drawings, the drawings are not necessarily drawn to scale unless specifically indicated.

The phrase "coupled to" is broad enough to refer to any suitable coupling or other form of interaction between two or more entities. Thus, two components may be coupled to each other even though they are not in direct contact with each other. The phrase "attached to" refers to interaction between two or more entities which are in direct contact with each other and/or are separated from each other only by a fastener of any suitable variety (e.g., an adhesive).

The terms "proximal" and "distal" are opposite directional terms. For example, the distal end of a device or component is the end of the component that is furthest from the practitioner during ordinary use. The proximal end refers to the opposite end (i.e., the end nearest the practitioner during ordinary use). The terms "proximal" and "distal" may also be used with reference to the respiratory tract. In such instances, where a first portion of the respiratory tract is distal of a second portion of the respiratory tract, the first portion of the respiratory tract is closer to the alveoli than the second portion of the respiratory tract. The term "nylon" generally has its ordinary meaning as understood by a skilled artisan, but does not encompass polyether block amides. Unless otherwise stated, all ranges include both endpoints and all numbers between the endpoints

The term stent, as used herein, is broad enough to refer to bare scaffold stents (such as bare metal stents) and covered stents (such as stents with a covering or coating, such as silicone layer across a portion of the scaffolding structure). Still further, though specific examples below may refer to use or features of covered stents, such disclosure is analogously applicable to bare metal stents and vice versa. Additionally, though specific examples may refer to stent configured for use in the airways, such disclosure may also be applied generally to stents used in other bodily lumens, such as vascular stents, vascular stent grafts, biliary stents, gastrointestinal stents, esophageal stents, and so forth.

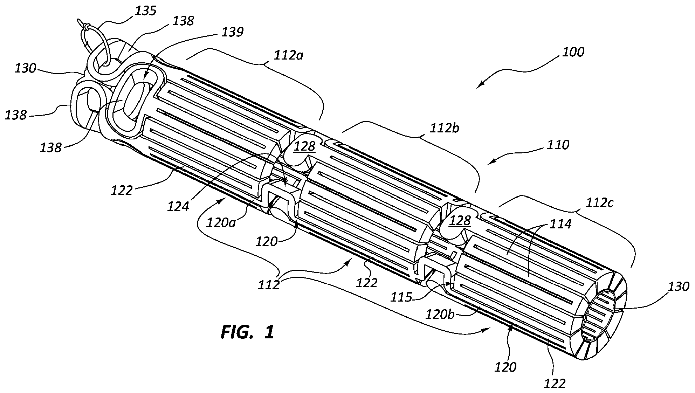

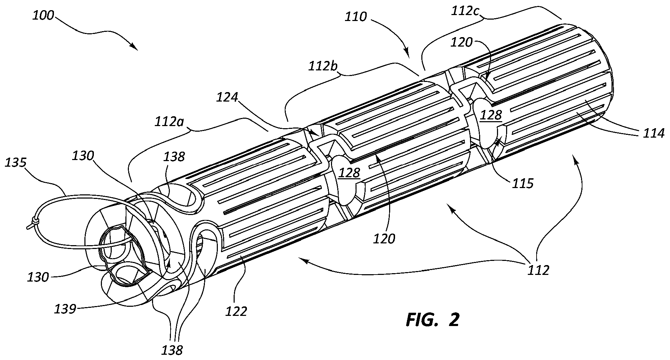

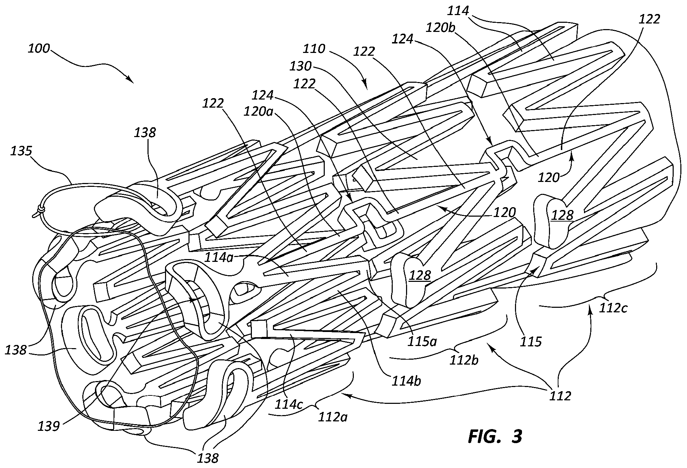

FIGS. 1 and 2 provide alternative perspective views of a stent 100 in a compressed (constrained) state, while FIG. 3 provides a perspective view of the same stent 100 in an expanded (unconstrained) state. As described herein, the stent 100 may be configured to transition from the compressed state to the expanded state when delivered into a patient, thereby supporting and/or opening a passageway within the patient.

As shown in FIGS. 1-3, the stent 100 includes a stent scaffold 110 and a coating 130. The stent scaffold 110 may be made from any suitable material. For example, in some embodiments, the stent scaffold 110 is made from a memory alloy, such as a nickel-titanium alloy. In some embodiments, the stent scaffold 110 is formed by cutting a pattern into a tube of material. For example, the stent scaffold 110 may be formed by laser cutting the tube and subsequently stretching and/or expanding the tube. In some embodiments, the tube from which the stent scaffold 110 is cut has a thickness of between 0.20 mm and 0.55 mm, such as between 0.35 and 0.45 mm

The stent scaffold 110 may have a plurality of annular rows 112 arranged along the longitudinal direction of the stent scaffold 110. Each annular row 112 may include interconnected strut arms 114. As shown in FIG. 3, the strut arms 114 are connected such that they form a zigzag pattern of alternating peaks and valleys when the stent scaffold 110 is in an expanded configuration. In some embodiments, adjacent strut arms 114 will form acute angles relative to each other when the stent 100 is in the expanded state. In the embodiment depicted in FIGS. 1-3, each annular row has 24 strut arms. (Some embodiments may have a different number of strut arms (e.g., 18 or 36 strut arms) Adjacent annular rows 112 may be coupled to each other by connectors 120.

The embodiment depicted in FIGS. 1-3 has three annular rows 112a, 112b, and 112c. Throughout this disclosure, particular examples of components may be designated by a reference numeral that is followed by a letter. For example, the reference numeral 112 refers generally to the annular rows of the stent scaffold 110. Specific annular segments are labeled 112a, 112b, and 112c. This pattern of identifying particular examples of general or repeating components may be used throughout this disclosure.

Each annular row 112a, 112b, 112c of the embodiment shown in FIGS. 1-3 includes multiple strut arms 114 that are arranged in a zigzag pattern when the stent 100 is in the expanded state. For example, as shown in FIG. 3, the strut arm 114a is coupled to the strut arm 114b such that the two arms 114a, 114b form a peak in the zigzag pattern. The strut arm 114b is further coupled to the strut arm 114c such that the two arms 114b, 114c form a valley in the zigzag pattern.

In the illustrated embodiment, adjacent strut arms 114, such as strut arms 114a, 114b are coupled at an apex, such as apex 115a. The angle formed at the apexes 115 by two adjacent strut arms 114 may be designed to provide the stent scaffold 110 with particular properties. In some embodiments, the angle formed at each apex 115 is between 20 degrees and 60 degrees, such as between 30 and 40 degrees between 35 and 45 degrees, or between 40 degrees and 50 degrees. In some embodiments, the struts of stents of relatively small diameter (e.g., between 5 and 7 mm) form angles of between 27 and 33 degrees. In some embodiments, the struts of stents having a somewhat larger diameter (e.g., between 9 and 15 mm) form angles of between 36 and 42 degrees.

Each strut arm 114 may define a length along the strut arm 114. Again, as shown in FIG. 3, each strut arm 114 is coupled to two other strut arms 114, thereby forming apexes 115 on both ends of the strut arm 114. The length of a single strut arm 114 is the length of the strut arm 114 from a first end to a second end, or the distance between each apex 115 at which the strut arm 114 is coupled to an adjacent strut arm 114.

The relative lengths of the strut arms 114 may affect the overall properties of the stent scaffold 110. For example, relatively longer strut arms 114 may result in a "softer" (meaning more compressible in a transverse direction) stent scaffold 110 than stents 110 where the strut arms 114 are relatively shorter. In some embodiments, the strut arms 114 have a length of between 2 mm and 5 mm. In some embodiments, the strut arms 114 of each annular row 112 are the same length as the strut arms 114 of each adjacent row 112. In other embodiments, the lengths of the strut arms 114 differ from row to row.

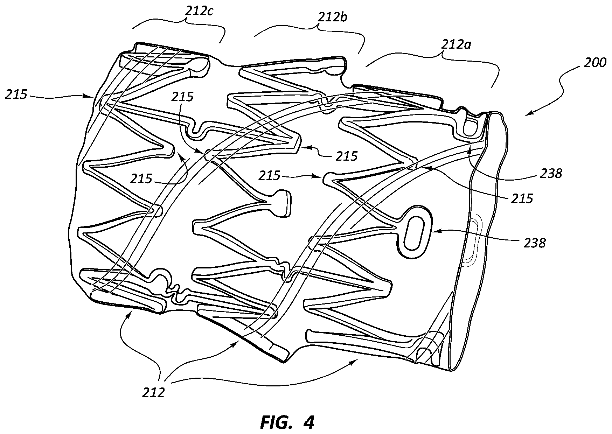

In certain embodiments, the strut arms 114 are straight or substantially straight when the stent scaffold 110 is in the expanded configurations. In other embodiments (e.g., FIGS. 4-6), the strut arms may be curved (or slightly curved) when the stent is in the expanded state (even when the strut arms are straight when the stent is in the compressed state). For example, a strut arm may be understood as having a first portion and a second portion. The first portion and the second portion may or may not be the same length. The strut arm generally may have an inflection point located between the first and second portions of the strut arm. Thus, the strut arm may be curved in the general shape of a sigmoid curve. In other words, the first portion of the strut arm forms a first roughly arcuate path, and the second portion of the strut arm forms a second roughly arcuate path. Thus, the strut arm can have a wave-like shape formed by the strut arm starting to curve in one direction, and then curving in a second direction. Accordingly, the strut arm has an "inflection point" at or around the point where the first portion meets the second portion.

In other embodiments, the strut arms may have a single curve or may resemble other types of curves when the stent is in the expanded state. Furthermore, while in some instances each strut arm may have a curved shape similar to the other strut arms of the stent, in other embodiments multiple strut arms on the same stent-including strut arms disposed in the same annular row-may have different shapes.

As shown in FIGS. 1-3, adjacent annular rows 112 may be coupled by connectors 120. In some embodiments, the connectors 120 may be coupled to the annular rows 112 at the apexes 115 formed by adjacent strut arms 114 In some embodiments, the connectors 120 are equally spaced around the circumference of the stent scaffold 110. In the embodiment of FIGS. 1-3, the connectors 120 are aligned circumferentially along the longitudinal direction of the stent scaffold 110. For instance, in some embodiments, a single apex 115 is coupled to two connectors 120, such as connectors 120a and 120b. In other embodiments, the connectors are offset in the circumferential direction. In such embodiments, any apex is coupled to no more than one connector. In some embodiments, some connectors are aligned while others are offset.

As shown in FIGS. 1-3, the connectors 120 may couple two adjacent annular rows 112 together. For instance, the connectors 120 may be coupled to each annular row 112 at apexes 115 on each annular row. Some connectors 120, such as the connectors 120a, 120b, may have one or more straight portions 122 and a non-linear (e.g., curved or bent) portion 124. For instance, in the illustrated embodiment, connector 120a includes two straight portions 122 and a non-linear portion 124 that is disposed at between the straight portions 122 such that the connector is positioned between adjacent annular rows 112 For the connector 120b, the straight portion 122 of the connector 120a spans much of the distance between the adjacent annular rows 112. The non-linear portion 124 of the connector 120b can adopt a curved, bent, rounded, square, or omega-shaped (Ω) configuration

The non-linear portions 124 of the connectors 120 may add flexibility and/or elasticity to the stent scaffold 110. For instance, an omega shape, having two ends relatively near each other connected by a curved or bent member (the round portion of the omega), may be configured to provide added flexibility to the stent scaffold 110. In some embodiments, the non-linear portions 124 may add axial strength to the stent scaffold 110. In some instances, axial strength may be desirable for expanding, contracting, repositioning, and/or removing a stent scaffold 110.

A first set of connectors 120a connects the first annular row 112a to the second annular row 112b, while a second set of connectors 120b couples the second annular row 112b to the third annular row 112c. Each set of connectors 120 may have any suitable number of connectors 120, such as between 1 and 10 connectors (e.g., between 1 and 4 connectors, between 3 and 5 connectors, between 4 and 6 connectors, between 5 and 8 connectors, or between 7 and 10 connectors). For instance, in the embodiment depicted in FIGS. 1-3, both the first set of connectors 120a and the second set of connectors 120b have four connectors. Other embodiments may include additional annular rows (e.g., a fourth annular row and/or a fifth annular row) and corresponding connectors to couple adjacent rows to each other. In some embodiments, each set of connectors 120 includes the same number of connectors 120. Stated differently, each annular row 112 may be connected to an adjacent annular row 112 by the same number of connectors 120. In other embodiments, a first set of connectors that couple a first annular row to a second annular row may differ in number from a second set of connectors that couple the second annular row to a third annular row. For example, the first set of connectors may include eight connectors for stiffness, while a second set of connectors includes only four connectors for increased flexibility.

In some embodiments, each peak or valley is attached to a connector 120. For example, an embodiment that includes 36 strut arms per annular row may have 18 connectors that connect an annular row to an adjacent annular row. An embodiment that includes 24 strut arms per annular row may have 12 connectors that connect the annular row to an adjacent annular row. And an embodiment that includes 18 strut arms can include nine connectors that connect the annular row to an adjacent annular row.

In the stent scaffold 110 shown in FIGS. 1-3, the adjacent annular rows 112 are aligned such that apexes 115 at the peaks of the zigzag pattern in annular row 112a are circumferentially aligned with apexes 115 at the valleys of the zigzag pattern of the adjacent annular row 112b. In the depicted embodiment, the first set of connectors 120a couples the two adjacent annular rows 112a, 112b by coupling to valley apexes 115 of annular row 112a and to peak apexes 115 of the annular row 112b. (As used herein, "peaks" refer to high points and "valleys" refer to low points, as measured from one end of the stent. Thus the coupling of the two segments via a "peak-to-peak" connection would, if viewed from the opposite orientation, be a "valley-to-valley" connection.)

The second annular row 112b is circumferentially aligned with the third annular row 112c such that the apexes 115 at the peaks of the second annular row 112b are circumferentially aligned with the peaks of the third annular row 112c. Stated differently, a second set of connectors 120b may couple the second annular row 112b to the third annular row 112c to align the peaks of the second annular row 112b with the peaks of the third annular row 112c. In this manner, the annular rows 112 are aligned such that the valleys of first annular row 112a are aligned with peaks from the second annular row 112b and the third annular row 112c.

Thus, in some embodiments, a stent scaffold 110 may be designed such that the peaks/valleys of adjacent annular rows 112 are circumferentially aligned. In other embodiments, the peaks/valleys of adjacent annular rows may be circumferentially offset. In some embodiments, adjacent rows may be positioned such that peaks of one row are circumferentially aligned with peaks of an adjacent row. In some embodiments, adjacent rows may be positioned such that peaks are circumferentially aligned with valleys. In some embodiments, the peaks of one row are circumferentially aligned with the peaks of one adjacent row and the valleys of another adjacent row.

It will be appreciated by one of skill in the art having the benefit of this disclosure, that in alternative embodiments any combination of alignment/non-alignment of peaks and valleys between any set of annular rows is within the scope of this disclosure.

The stent scaffold 110 of FIGS. 1-3 further includes generally rounded anti-migration portions 128 coupled to certain apexes 115. Stated differently, the one or more anti-migration portions 128 may include a bulbous end on a peak or valley of an annular row 112. (In other embodiments, the anti-migration portions lack a bulbous end, such as anti-migration portions that are formed from "normal" raised struts.) In some embodiments, the anti-migration portions 128 may be configured to contact the inside diameter of a body lumen, and thus restrict migration of the stent scaffold 110 within the body lumen. In certain embodiments, the anti-migration portions 128 may be positioned radially outward relative to the remainder of the stent scaffold 110. This arrangement allows the anti-migration portions 128 to engage the body lumen and minimize migration of the stent 100.

In some embodiments, the anti-migration portions 128 have a width of between 0.2 mm and 1.5 mm, such as between 0.9 mm and 1.2 mm. In the embodiment of FIGS. 1-3, each anti-migration portion 128 is disposed in a proximally oriented direction, and is thus configured to minimize migration of the stent scaffold 110 in the proximal direction when disposed within a lumen of a patient. In other embodiments, some or all of the anti-migration portions 128 may be disposed in a distally oriented direction.

The stent 100 of FIGS. 1-3 further includes a coating 130 that is coupled to the stent scaffold 110. The coating 130 may interact with the stent scaffold 110 to form a non-porous tubular structure that is open at both a proximal end of the stent scaffold 110 and a distal end of the stent scaffold 110. The coating 130 may define an inner portion of the stent 100. In some embodiments, the coating 130 is disposed on an inner (i.e., luminal) surface of the stent scaffold 110. The coating 130 may provide a smooth luminal surface. In some embodiments, the coating is disposed on an outer surface of the stent. The coating 130 may be elastomeric or polymeric, or may include any other suitable material. In some embodiments, the coating 130 includes polyurethane, while in certain embodiments, the coating 130 consists of polyurethane. In some embodiments, the coating 130 includes silicone, while in certain embodiments the coating 130 consists of silicone. In some embodiments the coating 130 may include multiple subparts or layers. In some embodiments, the coating 130 includes a low-friction surface. In some embodiments, the coating 130 may decrease or prevent tissue ingrowth. In some embodiments, the coating 130 may allow for tissue ingrowth. In some embodiments, the coating 130 includes a first layer of polyurethane that covers the inner and outer diameters of the stent 110 and a hydrophilic layer having a low coefficient of friction that is applied only to the inner surface of the polyurethane layer, only to the outer surface of the polyurethane layer, or to both the inner and outer surfaces of the polyurethane layer. In some embodiments, the coating 130 is a drug-eluting coating. Some embodiments may lack a coating. In other words, in some embodiments, a stent (but not a stent) is implanted within a patient.

In some embodiments, the stent scaffold 110 of the stent 100 may include one or more eyelets 138. The eyelets 138 may be designed to facilitate attachment of a suture 135 to the stent scaffold 110 or the stent 100.

In the depicted embodiment, each eyelet 138 includes an aperture 139. In some embodiments, the aperture 139 is elongate in the circumferential direction of the stent scaffold 110. Such a design may distribute the expansive force of the stent scaffold 110 acting on a body lumen when the stent scaffold 110 is deployed. The distribution of force, in connection with the smooth and rounded shape of the eyelets 138, may be configured to lessen the trauma to body tissue that contacts the ends of the stent scaffold 110.

In some embodiments, the eyelets 138 are positioned at or adjacent to the proximal end of the stent scaffold 110. For example, in the depicted embodiment, the eyelets 138 are positioned at the proximal end of the stent scaffold 110, but not at the distal end. Stated differently, in some instances, the stent 100 may be placed within a patient such that the eyelets 138 are positioned proximally of the remainder of the stent 100. Such placement of the eyelets 138 may facilitate removal of the stent 100 from the patient. In some embodiments, one or more eyelets may additionally or alternatively be disposed at or adjacent to the distal end of the stent scaffold 110. In some embodiments, eyelets 138 are positioned on each valley of the first row 112a. In other embodiments, eyelets are positioned at only a subset of the valleys of the first row 112a in a regular or irregular pattern.

In some embodiments, one or more sutures 135 are attached to one or more eyelets 138. For example, in the depicted embodiment, the single suture 135 (i.e., the only suture) is a loop-shaped suture that extends through one (and only one) eyelet 138 of the stent scaffold 110. Stated differently, the suture 135 shown in FIGS. 1-3 does not extend around the circumference of the stent 100. Such a suture 135 may be referred to as an "earring suture." In some embodiments, the loop-shaped suture 135 is disposed at or adjacent to the proximal end of the stent 100. The suture 135 may be attached to the stent scaffold 110 by passing one end of the suture 135 through the eyelet 138 and forming a knot to form a closed loop. An adhesive may be applied to the knot to prevent the knot from becoming undone. In other words, an adhesive may be used to prevent uncoupling of portions of the suture 135. Relative to other sutures 135 (such as a purse-string suture described below), the earring suture 135 may allow for easier insertion into a small channel. Stated differently, the earring suture 135 may have, relative to at least some other sutures, less bulk around the circumference of the stent scaffold 110 when the stent scaffold 110 is in a compressed configuration. This effect on bulk may be due to the fact less material may be used in the earring suture 135 than a purse-string suture that extends around the circumference. Additionally or alternatively, use of an earring suture due to the fact that, when compressed, the earring suture 135 may be longitudinally offset from the stent scaffold 110 structure, resulting in less material being compressed into a deployment pod at a particular longitudinal position. Such a suture 135 may additionally or alternatively, relative to at least some other sutures, reduce friction with adjacent surfaces. The suture 135 may, in some instances, also have less surface area to which particles, mucous, or other matter may accumulate. In some embodiments, a plurality of earring sutures 135 (e.g., two sutures) are used, wherein each suture 135 is attached to a different eyelet 138.

In some embodiments, a different type of suture may be used, such as a suture that extends through a plurality of eyelets to form a purse-string suture that, when pulled on by a practitioner, facilitates "necking down" of the stent. Generally, such sutures extend around the circumference of the stent. Such sutures are described in U.S. Appl. No. 13/153,150. In still other embodiments, no suture is used. The one or more sutures 135-whether earring sutures or purse-string sutures-may be designed to facilitate repositioning and/or removal of the stent 100, thereby allowing a practitioner to capture a deployed stent 100.

The suture(s) 135 may be formed of a metal, a thread, or any other suitable material. In some embodiments, the suture 135 includes or is made from polyethylene, such as ultra-high molecular weight polyethylene. In some embodiments, the suture 135 may comprise one or more radiopaque portions for use in deploying, removing or repositioning the stent scaffold 110. The radiopaque portions may be formed from a metal, such as gold, and enable a practitioner to more easily capture the suture 135 of the deployed stent scaffold 110 with a remote capturing tool. Similarly, the suture 135 may additionally or alternatively include endoscopic markers or markers visible through an endoscope or bronchoscope to aid a practitioner in viewing or manipulating the stent scaffold 110 in connection with an endoscope or bronchoscope. In some embodiments, certain markers, such as markers of gold, may be both radiopaque and visible through an endoscope or bronchoscope.

In some instances, the sutures 135 described herein is used to remove a stent scaffold 110 or stent 100 from a patient as described in greater detail below.

The stent 100 or stent scaffold 110 may be configured to transition from a compressed configuration (shown in FIGS. 1 and 2) to an expanded (uncompressed or unconstrained) configuration (shown in FIG. 3).

In the embodiment shown in FIGS. 1-3, the stent 100 and the stent scaffold 110 each have a diameter of approximately 10 mm and a length of approximately 15 mm when the stent 100 and the stent scaffold 110 are in the expanded configuration.

Other sizes of stents and stents are also within the scope of this disclosure. More broadly, in some embodiments, the stent, when in the compressed configuration, may be configured to be delivered through a channel (e.g., a channel of a bronchoscope) that has a diameter of 6 mm or less, 5 mm or less, 4 mm or less, 3.5 mm or less, 3.2 mm or less, 3.0 mm or less, or 2.8 mm or less. In some embodiments, the channel has a diameter of between 2.0 mm and 3.5 mm, such as a diameter of between 2.8 and 3.2 mm When compressed, the stent scaffold 110 or stent 100 may have a diameter of between 0.07" and 0.18", such as between 0.07" and 0.09", between 0.08" and 0.15", between 0.11" and 0.15", between 0.11" and 0.19", between 0.15" and 0.19". In some embodiments, the stent scaffold 110 or stent 100, is designed to be compressed to a size of between 0.005" and 0.020" below the size of the inner diameter of a corresponding deployment pod (described in greater detail below).

When unconstrained and in the expanded configuration, a stent may have a diameter of between 5 mm and 15 mm, such as between 5 mm and 14 mm, between 5 mm and 7 mm, between 6 mm and 8 mm, between 7 mm and 9 mm, between 8 mm and 10 mm, between 9 mm and 11 mm, between 10 mm and 12 mm, between 11 mm and 13 mm, between 12 mm and 14 mm, or between 13 mm and 15 mm. Stents having a relatively small diameter may be configured for delivery through a relatively narrow bronchoscope and for placement in a relatively narrow passageway relative to embodiments that have a larger diameter.

When unconstrained, the stent may have a length of between 5 mm and 40 mm, such as between 5 mm and 15 mm, between 5 mm and 8 mm, between 6 mm and 9 mm, between 7 mm and 10 mm, between 8 mm and 11 mm, between 9 mm and 12 mm (e.g., 10 mm), between 10 mm and 13 mm, between 11 mm and 14 mm, or between 12 mm and 15 mm.

In some embodiments, the stent is flared such that the proximal and/or distal ends of the stent have a larger diameter than a central region of the stent. In other embodiments, such as the embodiment shown in FIGS. 1-3, the stent scaffold 110 is not flared.

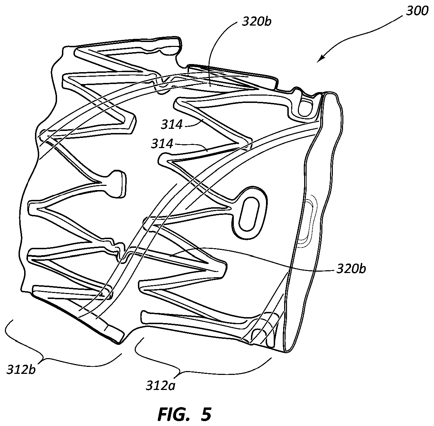

FIG. 4 depicts an embodiment of an expandable stent 200 that resembles the stent 100 described above in certain respects. Accordingly, like features are designated with like reference numerals, with the leading digits incremented to "2." For example, the embodiment depicted in FIG. 4 includes a coating 230 that may, in some respects, resemble the coating 130 of FIGS. 1-3. Relevant disclosure set forth above regarding similarly identified features thus may not be repeated hereafter. Moreover, specific features of the stent 100 and related components shown in FIGS. 1-3 may not be shown or identified by a reference numeral in the drawings or specifically discussed in the written description that follows. However, such features may clearly be the same, or substantially the same, as features depicted in other embodiments and/or described with respect to such embodiments. Accordingly, the relevant descriptions of such features apply equally to the features of the stent 200 and related components depicted in FIG. 4. Any suitable combination of the features, and variations of the same, described with respect to the stent 100 and related components illustrated in FIGS. 1-3 can be employed with the stent 200 and related components of FIG. 4, and vice versa. This pattern of disclosure applies equally to the embodiments depicted in FIGS. 5-6 and described hereafter, wherein the leading digits may be further incremented.

The stent 200 shown in FIG. 4 is generally analogous to the stent 100 shown in FIGS. 1-3, but differs from the stent 100 with respect to alignment of the apexes 215 in the annular rows 212. More particularly, for the stent 200, the peaks of the first annular row 212a (as viewed from the proximal end of the stent which contains the eyelets 238) are circumferentially aligned with the peaks of the second annular row 212b and the valleys of the third annular row 212c. In contrast, for the stent scaffold 110 of FIGS. 1-3, the peaks of the first annular row 112a (as viewed from the proximal end of the stent 100 which contains the eyelets 138) are circumferentially aligned with both the valleys of the second annular row 112b and the valleys of the third annular row 112c.

FIG. 5 provides a perspective view of an expandable stent 300 (in an expanded configuration) according to another embodiment. The stent 300 is generally analogous to the stents 100, 200 described above. However, instead of including three annular rows, the stent 300 includes only two annular rows 312a, 312b. Each annular row 312a, 312b includes 24 struts arms 314 that form a circumference of approximately 10 mm. The first annular row 312a is coupled to the second annular row 312b via four connectors 320b that are evenly spaced about the circumference of the stent 300. The peaks of the first annular row 312a are circumferentially aligned with the peaks of the second annular row 312b.

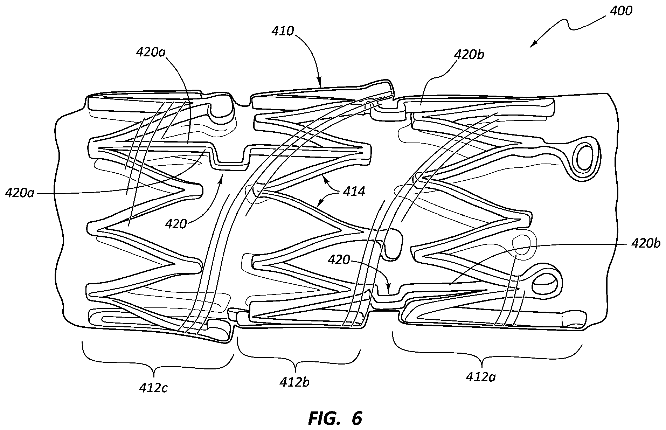

FIG. 6 provides a side view (or approximates a side view) of a stent 400 according to another embodiment, with the stent 400 in the expanded configuration. The stent 400, when in the expanded configuration, has a smaller diameter than stents 100, 200, 300 described above. For example, the stent 400 has a diameter of approximately 6 mm when in the expanded state. When in the compressed state, the stent 400 can have a diameter of between approximately 0.06" to 0.08". The stent 400 has a length of between 12 mm and 16 mm, such as 15 mm. Shorter stents (e.g., 10 mm stents) having a similar diameter can be formed by removing an annular row 412.

The stent 400 depicted in FIG. 6 includes a first annular row 412a, a second annular row 412b, and a third annular row 412c. Each row 412 has 18 strut arms 414 that together define the circumference of the stent scaffold 410. Each annular row 412 is connected to an adjacent annular row 412 via three connectors 420. For instance, the first annular row 412a is connected to the second annular row 412b via three connectors 420b. The second annular row 412b is connected to the third annular row 412c via a different set of three connectors 420a. The first set of connectors 420b is circumferentially offset from the second set of connectors 420a. The peaks of the first annular row 412a are circumferentially aligned with the peaks of the second annular row 412b and the valleys of the third annular row 412c.

The stent 400 depicted in FIG. 6 may be designed to travel through a working channel of a bronchoscope, such as therapeutic bronchoscope (e.g., Olympus BF-1TH190). The working channel may have a diameter of 3.2 mm or less, 3.0 mm or less, and/or 2.8 mm or less. In some embodiments, the stent 400, when in the collapsed state, can be disposed within a 7.9 Fr deployment pod.

Stents and stent having various other dimensions are within the scope of this disclosure. For example, stents of the following dimensions (or approximate dimensions) may be fashioned according to the principles described herein (the first dimension is the diameter while the second dimension is the length): 6 mm × 15 mm, 6 mm × 10 mm, 8 mm × 10 mm, 10 mm × 10 mm, 12 mm × 10 mm, or 14 mm × 10 mm.

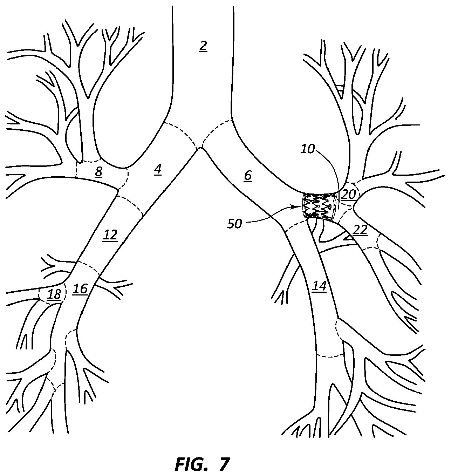

Stents of relatively small diameter, such as the stent 400 described in FIG. 6, may be used to treat strictures in relatively narrow passageways. Some passageways that may be treated with such stents are shown in FIG. 7, which shows the tracheobronchial tree of the respiratory tract. As shown in FIG. 7, the tracheobronchial tract includes the trachea 2, the right main bronchus 4, the left main bronchus 6, the right upper lobe 8, the left upper lobe 10, the intermediate bronchus 12, the left lower lobe 14, the right lower lobe 16, the middle lobe 18, the superior division bronchus, 20, and the lingular bronchus 22. Some stents, including stents described herein, may be used in the trachea 2, the right main bronchus 4, or the left main bronchus 6. Stents having a relatively smaller diameter may be used at more distal locations, such as the locations identified with numerals 8, 10, 12, 14, 16, 18, 20, and 22.

### Stent Delivery Device

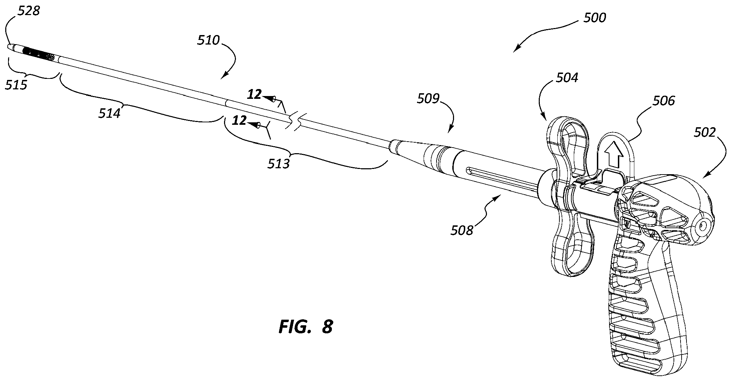

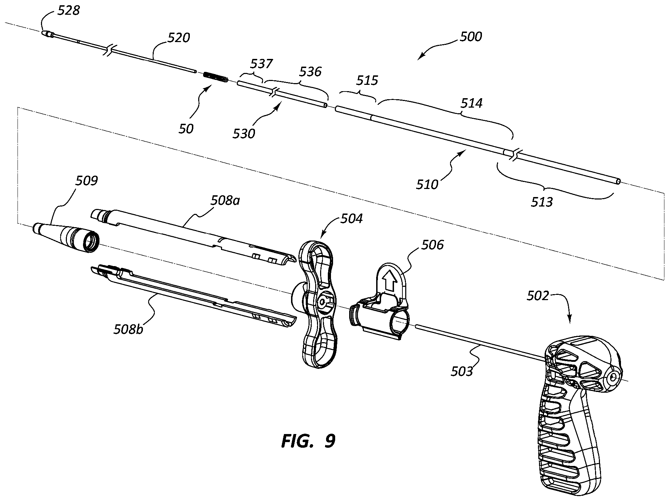

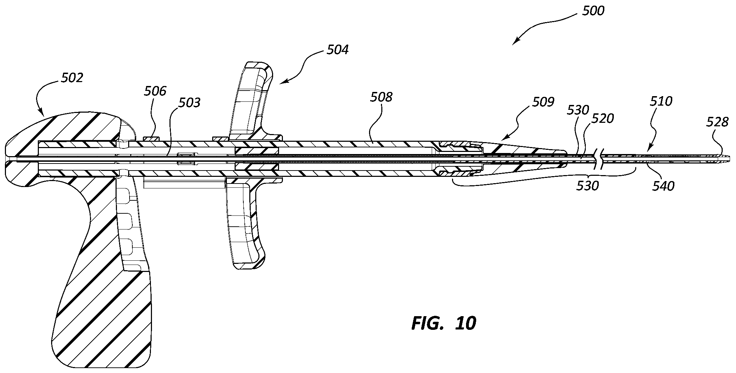

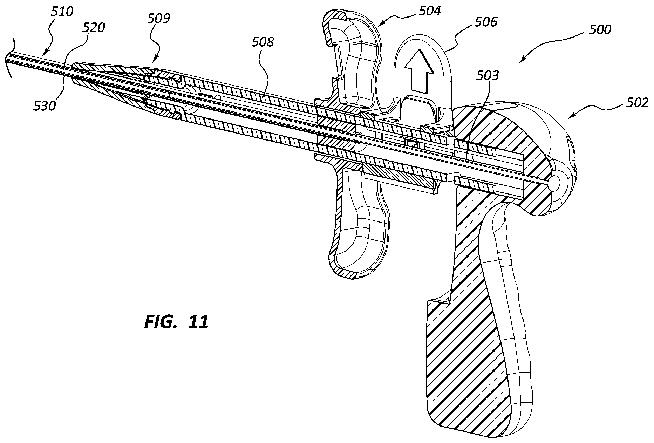

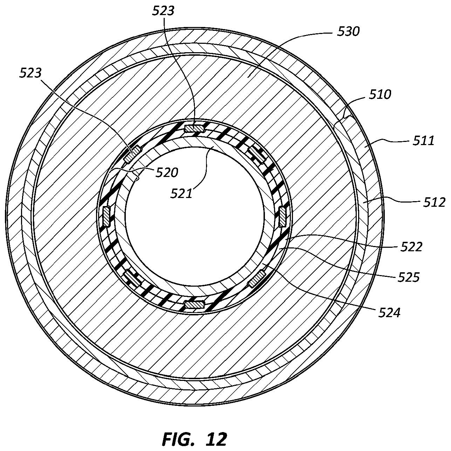

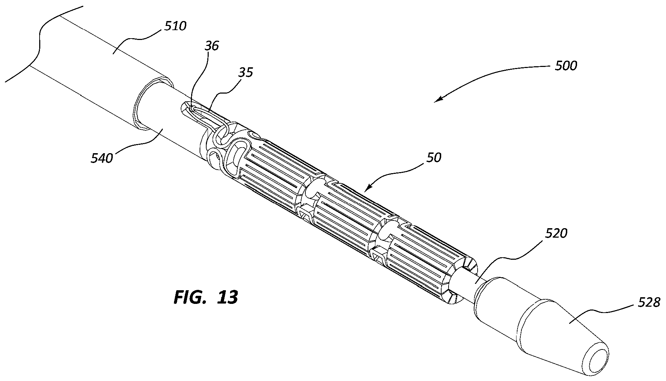

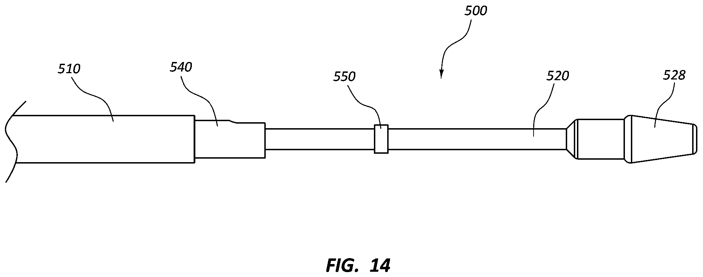

FIGS. 8-14 provide various views of a stent delivery device 500. More particularly, FIG. 8 provides a perspective view of the stent delivery device 500. FIG. 9 provides an exploded perspective view of the stent delivery device 500. FIG. 10 provides a cross-sectional side view of the stent delivery device 500. FIG. 11 provides a cross-sectional perspective view of the stent delivery device 500. FIG. 12 provides a cross-sectional view of a portion of the stent delivery device 500 through plane 12-12 of FIG. 8. FIG. 13 provides a perspective view of a distal portion of the stent delivery device 500. And FIG. 14 provides a side view of a portion of the stent delivery device 500.

As shown in FIGS. 8-14, the stent delivery device 500 includes a handle 502, an actuator 504, a lock 506, a support shaft 508, a tapered segment 509, an outer sheath 510, and a tubular shaft 520.

The handle 502 may be gripped by a practitioner using a single hand. In the depicted embodiment, the handle 502 includes an elongate shaft 503 that extends distally from the handle 502. In some embodiments, the elongate shaft 503 is formed from a metal or metal alloy. In other embodiments, the elongate shaft 503 is formed from a plastic material.

The support shaft 508 may be coupled (e.g., attached) to the handle 502 such that the support shaft 508 extends distally from the handle 502. The support shaft 508 may partially or completely enclose the elongate shaft 503 of the handle 502. In some embodiments, the support shaft 508 is formed from two separate pieces (e.g., halves) that are coupled to one another. For example, in some embodiments, two separate pieces of the support shaft 508 are coupled to one another (at least in part) via a tapered segment 509. In some embodiments, the length from the distal end of the tapered segment to 509 the distal end of the stent delivery device 500 is between 50 cm and 150 cm, such as between 65 cm 120 cm.

The actuator 504 may include an exterior region (e.g., a trigger) that is configured for direct contact with the finger(s) of a practitioner and an interior region that is disposed within the support shaft 508. The interior region of the actuator 504 may be coupled and/or attached (e.g., via an adhesive) to the outer sheath 510 such that actuation (e.g., retraction) of the actuator 504 causes the outer sheath 510 to be displaced proximally relative to the tubular shaft 520.

The outer sheath 510 may be coupled (e.g., attached) to the actuator 504 and extend distally therefrom. The outer sheath 510 may be configured to enclose a stent 50 when the stent delivery device 500 is in a first configuration and then to release the stent 50 when the stent delivery device 500 is in a different configuration. (The stent 50 may, in some circumstances, correspond with the stents and stents 100, 200, 300, 400 described above. In other embodiments, the stent 50 differs in some respect from such stents.)

In some embodiments, the outer sheath 510 includes a proximal portion 513, a distal portion 515, and an intermediate portion 514. The proximal portion 513 may be considered a "shaft portion," the intermediate portion 514 may be a "flex portion" and the distal portion 515 may be a "deployment pod" that is configured for crimping, contracting, or otherwise holding a stent 50 in a compressed state.

In some embodiments, the distal portion 515 is less flexible than the intermediate portion 514. In some embodiments, the distal portion 515 of the outer sheath 510 may include or be formed from a relatively hard and/or inflexible material, such as nylon (e.g., nylon 12). In contrast, the intermediate portion 514 of the outer sheath 510 may be formed from a more flexible material, such as PEBAX 7233. The relatively hard and/or inflexible distal portion 515 may be less prone to breakage or deformation arising from the expansive force provided by the stent 50 within the deployment pod. As the relatively hard and/or inflexible distal portion 515 does not expand to a significant extent as a result of the expansive force provided by the stent 50, the distal portion 515 may reduce the amount of friction between the distal portion 515 of the outer sheath 510 and a channel (e.g., a channel of a bronchoscope) into which the stent delivery device 500 is inserted.

In some embodiments, the distal portion 515 is transparent. The distal portion 515 of the outer sheath 510 may be configured to constrain the stent 50 in a compressed state. Stated differently, the outer sheath 510 may serve to constrain the stent 50 until the outer sheath 510 is retracted during deployment. In this manner, the distal portion 515 of the outer sheath 510 may define a deployment pod which is designed so that a stent 50 may be coaxially disposed within the enclosure formed by the distal portion 515 of the outer sheath 510. Stated differently, the stent 50 may be disposed between the tubular shaft 520 and the outer sheath 510.

As noted above, the intermediate portion 514 of the outer sheath 510 may be more flexible than the distal portion 515 of the outer sheath 510. The relative flexibility of the intermediate portion 514 may improve trackability of the stent delivery device 500 over a guidewire. The relative flexibility of the intermediate portion 515 may also render the stent delivery device 500 less traumatic upon insertion into a patient.

In some embodiments, the proximal portion 513 of the outer sheath 510 is harder and/or less flexible than the intermediate portion 514. In some embodiments, the proximal portion 513 of the outer sheath 510 comprises nylon (e.g., nylon 12). The relatively stiff proximal portion may resist kinking and/or facilitate axial displacement and/or torque transference of the outer sheath 510 and/or the stent delivery device 500.

1 inch corresponds to 2.54 cm, such conversion is applied to the following units. In some embodiments, the distal portion 515 is between 0.5 and 2 inches in length, the intermediate portion 514 is between 2 and 6 inches in length, and the proximal portion 513 is more than 18 inches in length. In some embodiments, the proximal portion 513 of the outer sheath 510 has a durometer of between 72 and 100 (or greater) on the Shore A scale. The intermediate portion 514 of the outer sheath 510 may have a durometer of between 60 and 70 on the Shore A scale, and the distal portion 515 may have a durometer of between 40 and 55 on the Shore A scale. In some embodiments, the durometer for these sections may differ somewhat from the ranges disclosed above.

The tubular shaft 520 may be at least partially disposed within the outer sheath 510 and coupled (e.g., attached) to the handle 502 of the stent delivery device 500. In some embodiments, the tubular shaft 520 is attached to the handle 502. For example, the tubular shaft 520 may be disposed within and attached to the elongate shaft 503 of the handle 502.

In some embodiments, the tubular shaft 520 defines a guidewire lumen. Stated differently, the tubular shaft 520 may be configured to accommodate a guide wire (e.g., a guide wire of 0.035" in diameter). The guidewire may be used to direct delivery of the stent 50 to the appropriate location. Stated differently, the guidewire may allow safe guidance of the stent delivery system 500 to the intended implant site while minimizing the risk of injury to a passageway of the patient.

The tubular shaft 520 may include a relatively low-friction inner (i.e., luminal) surface 521 (see FIG. 12). The low-friction inner surface 521 may facilitate displacement of the stent delivery device 500 along a guide wire over a relatively tortuous pathway, such as a pathway leading to a relatively distal region of a lung. In some embodiments, the inner surface 521 of the tubular shaft 520 includes a polytetrafluoroethylene-polyamide blend. The inner surface 521 may be configured to minimize friction with a guide wire disposed within the tubular shaft 520, thereby facilitating advancement and/or retraction of the stent delivery device 500 over the guidewire.

In some embodiments, the tubular shaft 520 includes an outer layer 522, such as an outer layer 522 that includes or consists of a polyether block amide, such as PEBAX. In some instances, one or more layers and/or support structures may be disposed between the outer layer and the inner layer. For example, some embodiments include a support structure 523 (e.g., a braided tube formed from a metal alloy) that is disposed between the inner layer 521 and the outer layer 522. In some embodiments, the support structure 523 is disposed between intermediate layers 524, 525, such as intermediate polyimide layers. The polyimide of the intermediate layers 524, 525 may provide relatively high tensile strength while maintaining a relatively low wall thickness. The support structure 523 may add kink resistance and increase the pushability and/or torque transference of the stent delivery device 500.

In some embodiments, a distal tip 528 may be disposed at the distal end of the tubular shaft 520. For instance, in some embodiments, the distal tip 528 is attached to the tubular shaft 520 via overmolding. More specifically, in some embodiments, a polyether block amide tip may be overmolded onto the polyether block amide outer layer 522 of the tubular shaft 520. In some embodiments, the distal tip 528 is made from PEBAX 7233. In some embodiments, distal tip 528 is rigid enough to permit advancement through strictures of a passageway. In some embodiments, the distal tip 528 may include a generally frustoconical portion that tapers toward the distal end. In some embodiments, the distal tip 528 includes a radiopaque marker. For example, in some embodiments, a radiopaque material (e.g., barium) is distributed throughout the polymer used to form the distal tip 528. In other embodiments, a band (e.g., a platinum and/or iridium band) or other marker is swaged or crimped over the tubular shaft 520 and then the distal tip 528 is subsequently overmolded onto the tubular shaft 520. In other embodiments, the band or other marker is attached to an exterior portion of the distal tip 528. The use of radiopaque material may enable visualization of the distal tip 528 under fluoroscopic conditions.

In some embodiments, the stent delivery device 500 may further comprise a pliant anchor 550 disposed around an exterior of the tubular shaft 520. Stated differently, in some embodiments, the pliant anchor 550 is disposed around a circumference of the tubular shaft 520.

The pliant anchor 550 may be configured for engagement with the compressed stent 50. Stated another way, the pliant anchor 550 may at least partially grip, anchor, hold, and/or grasp the stent 50. In certain embodiments, the stent 50 may be disposed around the pliant anchor 550 and then the stent 50 may be constrained, crimped, and/or loaded around the pliant anchor 550. Further, a portion of the loaded stent 50 (e.g., an inner surface of the stent 50) may imprint into the pliant anchor 550.

In some embodiments, the pliant anchor 550 may be configured to limit or prevent longitudinal displacement of the constrained stent 50. For example, the pliant anchor 550 may grip the constrained stent 50 such that longitudinal displacement of the constrained stent 50 within the deployment pod is limited or prevented. In certain embodiments, the pliant anchor 550 may be configured to limit or prevent the constrained stent 50 from collapsing or longitudinally folding on itself. For example, the pliant anchor 550 may provide axial support to the constrained stent 50. Further, the pliant anchor 550 may be configured to partially surround one or more portions of the constrained stent 50, meaning that the pliant anchor 550 may conform to at least a portion of the constrained stent 50. For example, the pliant anchor 550 may conform to portions of the inner surface, shape, edges, and/or texture of the constrained stent 50. In this manner, the constrained stent 50 (e.g., the inner surface of the constrained stent 50) may at least partially imprint around the pliant anchor 550. In some embodiments, imprinting of a stent 50 around the pliant anchor 550 may support on or more rows of the stent 50.

The pliant anchor 550 can be formed from one or more materials that are flexible, malleable, moldable, pliable, and/or supple. For example, the pliant member 290 may comprise one or more silicones, polyolefins, polyether block amides (e.g., PEBAX^{®}), thermoplastic elastomers (e.g., CHRONOPRENE^{™}), and/or other suitable materials. In some embodiments, such as some embodiments where the pliant anchor 550 comprises a polyether block amide (e.g., PEBAX^{®}), the plaint anchor 550 is overmolded onto a tubular shaft 520 that has an outer layer 522 that is made from a polyether block amide. The pliant anchor 550 may be applied to or disposed on the tubular shaft 520 via any suitable technique, such as heat shrinking.

In some embodiments, an intermediate sheath 530 is disposed between the tubular shaft 520 and the outer sheath 510. For instance, in the depicted embodiment, the intermediate sheath 530 is disposed around the tubular shaft 520 and extends distally from the distal tip of the elongate shaft 503 of the handle 502.

The intermediate sheath 530 may have a proximal portion 536 and a distal portion 537. In some embodiments, the distal portion 537 of the intermediate sheath 530 is softer and/or more flexible than the proximal portion 536 of the intermediate sheath. In some embodiments, the distal portion 537 of the intermediate sheath 530 extends proximally from a position that is between 0.5 inches and 2.5 inches from the distal end of the stent delivery device 500. In some embodiments, the distal portion 537 of the intermediate sheath 530 has a length of between 1 inch and 5 inches. The proximal portion 536 of the intermediate sheath 530 may extend proximally from the distal portion 537 of the intermediate sheath 530. In some embodiments, the proximal portion 536 of the intermediate sheath 530 and the distal portion 537 of the intermediate sheath are attached to or integrally formed with one another. In other embodiments, the proximal portion 536 of the intermediate sheath 530 and the distal portion 537 of the intermediate sheath are separate structures.

Some embodiments further include a notched tubular segment 540 that is disposed distal of the distal portion 537 of the intermediate sheath 530. The notched tubular segment 540 may be disposed between the tubular shaft 520 and the outer sheath 510 when the stent delivery device 500 is configured for insertion into a patient. The notch in the notch tubular segment 540 may be configured to accommodate a knot 36 of a suture 35 (e.g., a looped suture). For example, as shown in FIG. 13, a portion of the suture 35 (e.g., the knot 36) may be disposed axial of (e.g., proximal of) the stent 50 such that the knot of the suture 35 is disposed within the notch of the notched tubular segment 540. Placement of the knot 36 of the suture 35 within the notch may reduce or minimize friction between the knot 36 and the outer sheath 510. More specifically, in some embodiments, the knot 36 is positioned such that the knot 36 is not disposed radially outward of the stent 50, and therefore does not stack on top of the stent 50 to increase friction with the outer sheath 510. In some embodiments, the notched tubular segment 540 comprises radiopaque material. In some embodiments, the notched tubular segment 540 is attached to or integrally connected with the distal portion 537 of the intermediate sheath 530. In other embodiments, the notched tubular segment 540 is a separate component. In some embodiments, the intermediate sheath 530 and the notched tubular segment 540 are not displaceable (or cannot be displaced more than a few millimeters) relative to the tubular shaft 520. Stated differently, in some embodiments, the intermediate sheath 530 and/or the notched tubular segment 540 are not displaceable longitudinally along the tubular shaft 520.

The lock 506 may be configured to prevent inadvertent displacement of the actuator 504. For instance, the lock 506 may be positioned between the actuator 504 and the handle, thereby preventing displacement (e.g., retraction) of the actuator 504 relative to the tubular shaft 520. When the practitioner is ready to retract the actuator 504 to deploy the stent 50, the lock 506 may be removed by pulling on the lock 506 in an upward direction. Once removed, the practitioner is free to retract the actuator 504 and deploy the stent 50.

### Deployment of Stent

To implant a stent 50 into a patient, a practitioner may obtain a stent delivery device, such as the stent delivery device 500 described above. A stent 50 may be disposed within a deployment pod of the stent delivery device 500. For example, a stent 50 may be crimped, compacted, or otherwise compressed around the tubular shaft 520 such that the stent 50 is disposed within a distal portion 515 of the outer sheath 510. Then, a portion of the delivery device 500 may be inserted through the nose or mouth of a patient, down the trachea, and into a region (e.g., the lung) of the patient. For instance, a distal tip 528 of the delivery device 500 may be inserted into and advanced within a channel of a bronchoscope to position the distal tip 528 of the stent delivery device adjacent to a stricture within the patient.

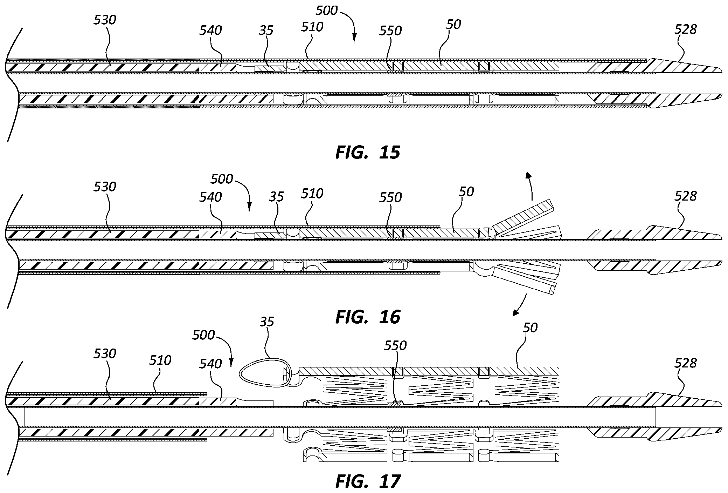

Once the distal end of the stent delivery device 500 is properly positioned (e.g., as shown in FIG. 15), the practitioner may manipulate the stent delivery device 500 to deploy the stent 50 from the deployment pod of the stent delivery device 500. For instance, in some embodiments, the practitioner may retract the actuator 504 relative to the handle 502. Actuation of the actuator 504 may cause the outer sheath 510 to be retracted (i.e., proximally displaced) relative to the tubular shaft 520 that is disposed within the outer sheath 510.

As the outer sheath 510 is initially retracted, a distal portion of the stent 50 may begin to expand while a proximal portion of the stent 50 remains in a compressed configuration as shown in FIG. 16. When in the position shown in FIG. 16 (i.e., with the outer sheath 510 partially retracted), the anchor 550 may prevent the stent 50 from prematurely "jumping" out of the deployment pod. Stated differently, until the outer sheath 510 has been retracted such that the distal end of the outer sheath is disposed proximal of the anchor 550, the anchor 550 may prevent complete deployment of the stent 50. In some embodiments, one or more indicia on the stent delivery device 500 (e.g., on the support shaft 508) may indicate when the actuator 504 has been sufficiently displaced such that the stent 50 begins to separate from the anchor 550. Stated differently, in some embodiments, the stent 50 remains constrained by the stent delivery device 500 until the actuator 504 is pulled beyond indicia disposed between the actuator 504 and the handle 502. This feature may allow for repositioning of the stent 50. In some circumstances, the procedure may be aborted and the entire stent delivery system 500 can be withdrawn at any time before the actuator 504 is pulled beyond the indicium located between the actuator 504 and the handle 502.

As shown in FIG. 17, further retraction of the actuator 504 may allow the stent 50 to separate from the anchor 550 and allow for deployment of the stent 50 into a passageway of the patient. Stated differently, proximal displacement of the actuator 504 may allow the stent 50 to expand to contact with and provide support for a passageway within the patient.

In some embodiments, the stent delivery device 500 is manipulated such that the stent 50 is deployed within a portion of the lung that is distal of either the left main bronchus or the right main bronchus. In other embodiments, the stent delivery device 500 is manipulated to deploy the stent 50 at some other location within the patient.

### Removal of Repositioning of Stent

In some circumstances, a practitioner may desire to remove or reposition a stent 50. To remove or reposition a stent 50 from a passageway of a patient, the practitioner may first engage a suture 35 of the stent 50. More specifically, a practitioner may grasp and displace the suture 35 through use of a remote access tool, such as grasping forceps. Retraction of the suture 35 in a proximal direction may allow for withdrawal or repositioning of the stent 50.

In some embodiments, the suture 35 is a loop-shaped suture, such as an earring suture. In some embodiments, the loop-shaped suture 35 is the only suture of the stent 50. In other embodiments, loop-shaped sutures 35 are attached to a plurality of eyelets of a stent 50.

Any methods disclosed herein include one or more steps or actions for performing the described method which is not part of the claimed invention. The method steps and/or actions may be interchanged with one another. In other words, unless a specific order of steps or actions is required for proper operation of the embodiment, the order and/or use of specific steps and/or actions may be modified. Moreover, sub-routines or only a portion of a method described herein may be a separate method within the scope of this disclosure. Stated otherwise, some methods may include only a portion of the steps described in a more detailed method.

Reference throughout this specification to "an embodiment" or "the embodiment" means that a particular feature, structure, or characteristic described in connection with that embodiment is included in at least one embodiment. Thus, the quoted phrases, or variations thereof, as recited throughout this specification are not necessarily all referring to the same embodiment.

Similarly, it should be appreciated by one of skill in the art with the benefit of this disclosure that in the above description of embodiments, various features are sometimes grouped together in a single embodiment, figure, or description thereof for the purpose of streamlining the disclosure. This method of disclosure, however, is not to be interpreted as reflecting an intention that any claim requires more features than those expressly recited in that claim. Rather, as the following claims reflect, inventive aspects lie in a combination of fewer than all features of any single foregoing disclosed embodiment. This disclosure includes all permutations of the independent claims with their dependent claims.

## Claims

1. A stent (100) comprising:
a plurality of annular rows (112), wherein each row of the plurality of annular rows (112) forms a zigzag pattern of alternating peaks and valleys; and
a plurality of connectors (120), wherein each connector (120) of the plurality of connectors couples an annular row (112) of the plurality of annular rows to an adjacent row of the plurality of annular rows;
wherein the stent (100) is configured to transition from a compressed configuration to an expanded configuration; and
wherein the stent (100), when compressed, is configured to be delivered through a channel that has a diameter of 3.2 mm or less;
**characterized in that**,
the stent (100) further comprises one or more eyelets (138) disposed on a proximal end of the stent (100); and
a loop-shaped suture (135) disposed through only one of the one or more eyelets (138) and not extending around a circumference of the stent (100), wherein the loop-shaped suture (135) is configured to facilitate removal of the stent (100) from a patient.

2. The stent of claim 1, wherein the stent (100), when in the compressed configuration, is configured to be delivered through a channel that has a diameter of 3.0 mm or less.

3. The stent of any one of claims 1 or 2, wherein the stent (100) has a diameter of between 5 mm and 14 mm when unconstrained and in the expanded configuration.

4. The stent of any one of claims 1 to 3, wherein the stent (100), when unconstrained, has a length of between 5 mm and 40 mm.

5. The stent of any one of claims 1 to 4, wherein the plurality of annular rows (112) comprise a first annular row and a second annular row, wherein a first set of connectors (120) of the plurality of connectors couple the first annular row to the second annular row.

6. The stent of any one of claims 1 to 5, wherein each one of the one or more eyelets (138) comprises an elongate aperture (139) oriented in a circumferential direction of the stent.

7. The stent of any one of claims 1 to 6, further comprising one or more anti-migration portions (128).

8. The stent of any one of claims 1 to 7, further comprising:
a coating (130) that interacts with the stent (100) to form a non-porous tubular structure that is open at a proximal end of the stent and a distal end of the stent.

9. A stent delivery device (500) comprising: a stent (100) of any of claims 1 to 8;
an actuator (504);
an outer sheath (510) comprising a proximal portion, a distal portion, and an intermediate portion, wherein the distal portion defines a deployment pod that is configured to accommodate the stent in a compressed state, wherein the deployment pod is less flexible than the intermediate portion;
a tubular shaft (520) at least partially disposed within the outer sheath (510), wherein the tubular shaft (520) defines a guidewire lumen;
wherein the outer sheath (510) is configured to be displaced proximally relative to the tubular shaft (520) in response to actuation of the actuator (504).

10. The stent delivery device (500) of claim 9, further comprising a lock (506) that prevents displacement of the outer sheath (510) relative to the tubular shaft (520).

11. The stent delivery device (500) of any one of claims 9 or 10, further comprising a pliant anchor (550) disposed around an exterior of the tubular shaft (520), wherein the pliant anchor (550) is configured for engagement with the compressed stent (100).

12. The stent delivery device (500) of any one of claims 9 to 11, further comprising a distal tip (528) that is attached to the tubular shaft (520).

13. The stent delivery device (500) of any one of claims 9 to 12, further comprising a notched tubular segment (540) disposed between the tubular shaft (520) and the outer sheath (510), wherein the notched segment (540) is configured to accommodate a knot of the loop-shaped suture (135).

## Patentansprüche

1. Stent (100), umfassend:
eine Vielzahl von ringförmigen Reihen (112), wobei jede Reihe der Vielzahl von ringförmigen Reihen (112) ein Zickzackmuster abwechselnder Spitzen und Täler bildet; und
eine Vielzahl von Verbindern (120), wobei jeder Verbinder (120) der Vielzahl von Verbindern eine ringförmige Reihe (112) der Vielzahl von ringförmigen Reihen an eine benachbarte Reihe der Vielzahl von ringförmigen Reihen koppelt;
wobei der Stent (100) dazu konfiguriert ist, aus einer zusammengedrückten Konfiguration in einer ausgedehnte Konfiguration überzugehen; und
wobei der Stent (100), wenn er zusammengedrückt ist, dazu konfiguriert ist, durch einen Kanal eingeführt zu werden, der einen Durchmesser von 3,2 mm oder weniger aufweist;
**dadurch gekennzeichnet, dass**
der Stent (100) ferner Folgendes umfasst: eine oder mehrere Ösen (138), die an einem proximalen Ende des Stents (100) angeordnet sind; und
einen schlingenförmigen Faden (135), der durch nur eine der einen oder der mehreren Ösen (138) hindurch angeordnet ist und sich nicht um einen Umfang des Stents (100) erstreckt, wobei der schlingenförmige Faden (135) dazu konfiguriert ist, das Entfernen des Stents (100) aus einem Patienten zu erleichtern.

2. Stent nach Anspruch 1, wobei der Stent (100), wenn er sich in der zusammengedrückten Konfiguration befindet, dazu konfiguriert ist, durch einen Kanal eingeführt zu werden, der einen Durchmesser von 3.0 mm oder weniger aufweist.

3. Stent nach einem der Ansprüche 1 oder 2, wobei der Stent (100) einen Durchmesser von zwischen 5 mm und 14 mm aufweist, wenn er nicht eingezwängt ist und sich in der ausgedehnten Konfiguration befindet.

4. Stent nach einem der Ansprüche 1 bis 3, wobei der Stent (100), wenn er nicht eingezwängt ist, eine Länge von zwischen 5 mm und 40 mm aufweist.

5. Stent nach einem der Ansprüche 1 bis 4, wobei die Vielzahl von ringförmigen Reihen (112) eine erste ringförmige Reihe und eine zweite ringförmige Reihe umfasst, wobei ein erster Satz von Verbindern (120) der Vielzahl von Verbindern die erste ringförmige Reihe an die zweite ringförmige Reihe koppelt.

6. Stent nach einem der Ansprüche 1 bis 5, wobei jede der einen oder der mehreren Ösen (138) eine langgestreckte Öffnung (139) umfasst, die in einer Umfangsrichtung des Stents orientiert ist.

7. Stent nach einem der Ansprüche 1 bis 6, ferner umfassend einen oder mehrere Antimigrationsabschnitte (128).

8. Stent nach einem der Ansprüche 1 bis 7, ferner umfassend:
eine Beschichtung (130), die mit dem Stent (100) interagiert, um eine nicht poröse röhrenförmige Struktur zu bilden, die an einem proximalen Ende des Stents und einem distalen Ende des Stents offen ist.

9. Stenteinführvorrichtung (500), umfassend:
einen Stent (100) nach einem der Ansprüche 1 bis 8;
einen Aktor (504);
eine äußere Hülle (510), umfassend einen proximalen Abschnitt, einen distalen Abschnitt und einen mittleren Abschnitt, wobei der distale Abschnitt eine Einbringkapsel definiert, die dazu konfiguriert ist, den Stent in einem zusammengedrückten Zustand aufzunehmen, wobei die Einbringkapsel weniger biegsam ist als der mittlere Abschnitt;
einen röhrenförmigen Schaft (520), der mindestens teilweise in der äußeren Hülle (510) angeordnet ist, wobei der röhrenförmige Schaft (520) ein Führungsdrahtlumen definiert;
wobei die äußere Hülle (510) dazu konfiguriert ist, als Reaktion auf die Betätigung des Aktors (504) relativ zu dem röhrenförmigen Schaft (520) proximal verschoben zu werden.

10. Stenteinführvorrichtung (500) nach Anspruch 9, ferner umfassend eine Verriegelung (506), die die Verschiebung der äußeren Hülle (510) relativ zu dem röhrenförmigen Schaft (520) verhindert.

11. Stenteinführvorrichtung (500) nach einem der Ansprüche 9 oder 10, ferner umfassend einen nachgiebigen Anker (550), der um ein Äußeres des röhrenförmigen Schafts (520) angeordnet ist, wobei der nachgiebige Anker (550) zum Eingriff mit dem zusammengedrückten Stent (100) konfiguriert ist.

12. Stenteinführvorrichtung (500) nach einem der Ansprüche 9 bis 11, ferner umfassend eine distale Spitze (528), die an dem röhrenförmigen Schaft (520) angerbracht ist.

13. Stenteinführvorrichtung (500) nach einem der Ansprüche 9 bis 12, ferner umfassend ein gekerbtes röhrenförmiges Segment (540), das zwischen dem röhrenförmigen Schaft (520) und der äußeren Hülle (510) angeordnet ist, wobei das gekerbte Segment (540) dazu konfiguriert ist, einen Knoten des schlingenförmigen Fadens (135) aufzunehmen.

## Revendications

1. Endoprothèse (100) comprenant :
une pluralité de rangées annulaires (112), dans laquelle chaque rangée de la pluralité de rangées annulaires (112) forme un motif de zigzag alternant crêtes et creux ; et
une pluralité de connecteurs (120), dans laquelle chaque connecteur (120) de la pluralité de connecteurs couple une rangée annulaire (112) de la pluralité de rangées annulaires à une rangée adjacente de la pluralité de rangées annulaires ;
dans laquelle l'endoprothèse (100) est configurée pour effectuer une transition d'une configuration comprimée à une configuration déployée ; et
dans laquelle l'endoprothèse (100), quand elle est comprimée, est configurée pour être délivrée à travers un canal qui a un diamètre de 3,2 mm ou moins ;
**caractérisé en ce que**,
l'endoprothèse (100) comprend en outre un ou plusieurs oeillets (138) disposés sur une extrémité proximale de l'endoprothèse (100) ; et
une suture en forme de boucle (135) disposée à travers seulement un des un ou plusieurs oeillets (138) et ne s'étendant pas autour d'une circonférence de l'endoprothèse (100), dans laquelle la suture en forme de boucle (135) est configurée pour faciliter le retrait de l'endoprothèse (100) d'un patient.

2. Endoprothèse selon la revendication 1, dans laquelle l'endoprothèse (100), quand elle est dans la configuration comprimée, est configurée pour être délivrée à travers un canal qui a un diamètre de 3,0 mm ou moins.

3. Endoprothèse selon l'une quelconque des revendications 1 ou 2, dans laquelle l'endoprothèse (100) a un diamètre compris entre 5 mm et 14 mm quand elle n'est pas contrainte et dans la configuration déployée.

4. Endoprothèse selon l'une quelconque des revendications 1 à 3, dans laquelle l'endoprothèse (100), quand elle n'est pas contrainte, a une longueur comprise entre 5 mm et 40 mm.

5. Endoprothèse selon l'une quelconque des revendications 1 à 4, dans laquelle la pluralité de rangées annulaires (112) comprennent une première rangée annulaire et une deuxième rangée annulaire, dans laquelle un premier ensemble de connecteurs (120) de la pluralité de connecteurs couple la première rangée annulaire à la deuxième rangée annulaire.

6. Endoprothèse selon l'une quelconque des revendications 1 à 5, dans laquelle chacun des un ou plusieurs oeillets (138) comprend une ouverture allongée (139) orientée dans une direction circonférentielle de l'endoprothèse.

7. Endoprothèse selon l'une quelconque des revendications 1 à 6, comprenant en outre une ou plusieurs partie anti-migration (128).

8. Endoprothèse selon l'une quelconque des revendications 1 à 7, comprenant en outre :
un revêtement (130) qui interagit avec l'endoprothèse (100) pour former une structure tubulaire non poreuse qui est ouverte au niveau d'une extrémité proximale de l'endoprothèse et d'une extrémité distale de l'endoprothèse.

9. Système de délivrance d'endoprothèse (500) comprenant :
une endoprothèse (100) selon l'une quelconque des revendications 1 à 8 ;
un actionneur (504) ;
une gaine externe (510) comprenant une partie proximale, une partie distale, et une partie intermédiaire, dans lequel la partie distale définit un fuseau de déploiement qui est configuré pour accueillir l'endoprothèse dans un état comprimé, dans lequel le fuseau de déploiement est moins flexible que la partie intermédiaire ;
un arbre tubulaire (520) disposé au moins partiellement au sein de la gaine externe (510), dans lequel l'arbre tubulaire (520) définit une lumière de fil-guide ;
dans lequel la gaine externe (510) est configurée pour être déplacée de manière proximale par rapport à l'arbre tubulaire (520) en réponse à l'actionnement de l'actionneur (504).

10. Dispositif de délivrance d'endoprothèse (500) selon la revendication 9, comprenant en outre un élément de verrouillage (506) qui empêche le déplacement de la gaine externe (510) par rapport à l'arbre tubulaire (520).

11. Dispositif de délivrance d'endoprothèse (500) selon l'une quelconque des revendications 9 ou 10, comprenant en outre une ancre pliante (550) disposée autour d'un extérieur de l'arbre tubulaire (520), dans lequel l'ancre pliante (550) est configurée pour une entrée en prise avec l'endoprothèse comprimée (100).

12. Dispositif de délivrance d'endoprothèse (500) selon l'une quelconque des revendications 9 à 11, comprenant en outre une pointe distale (528) qui est attachée à l'arbre tubulaire (520).

13. Dispositif de délivrance d'endoprothèse (500) selon l'une quelconque des revendications 9 à 12, comprenant en outre un segment tubulaire cranté (540) disposé entre l'arbre tubulaire (520) et la gaine externe (510), dans lequel le segment cranté (540) est configuré pour accueillir un noeud de la suture en forme de boucle (135).
